# EUROPEAN PATENT APPLICATION

(11) **EP 4 393 479 A1**
(43) Date of publication of application: **03.07.2024**
(21) Application number: 22216778.5
(22) Date of filing: 27.12.2022
(51) Int. Cl.: A61K 9/06, A61K 47/32, A61K 31/702, A61P 29/00

(54) **MANNURONATE, GULURONATE AND GULUMANNURONATE TOPICAL GEL OR CREAM AND METHOD FOR ITS PREPARATION**

(71) Applicant: Mirshafiey, Abbas, 14155 Teheran (IR); Lalander, Jacob, 1001 Riga (LV)
(72) Inventor: MIRSHAFIEY, Abbas, 14155 Teheran (IR); LALANDER, Jacob, LV-1001 Riga (LV)
(74) Representative: Maiwald GmbH

(57) **Abstract**

The present invention relates to gel or cream formulations for topical use, the formulation comprising β-D-mannuronic acid or α-L-guluronic acid or a mixture of β-D-mannuronic acid and α-L-guluronic acid, oligomers thereof, or pharmaceutically acceptable salts thereof, wherein the total amount of β-D-mannuronic acid or α-L-guluronic acid or the mixture of β-D-mannuronic acid and α-L-guluronic acid, oligomers thereof, or pharmaceutically acceptable salts thereof is 0.5 g to 40 g based on 100 g gel or cream formulation. Additionally, the invention relates to gel or cream formulations for topical use in a method for treating inflammation and/or any inflammatory reaction, and/or pain, and methods for preparing the gel or cream formulations.

## Description

### FIELD OF THE INVENTION

The present invention relates to gel or cream formulations for topical use, the formulation comprising β-D-mannuronic acid or α-L-guluronic acid or a mixture of β-D-mannuronic acid and α-L-guluronic acid, oligomers thereof, or pharmaceutically acceptable salts thereof, wherein the total amount of β-D-mannuronic acid or α-L-guluronic acid or the mixture of β-D-mannuronic acid and α-L-guluronic acid, oligomers thereof, or pharmaceutically acceptable salts thereof is 0.5 g to 40 g based on 100 g gel or cream formulation. Additionally, the invention relates to gel or cream formulations for topical use in a method for treating inflammation and/or any inflammatory reaction, and/or pain, and methods for preparing the gel or cream formulations.

### BACKGROUND OF THE INVENTION

Mannuronic acid and guluronic acid are natural uronic acids which are isolated and purified from various forms of alginates, which are polymers used as a thickener, binder, gelling agent or lubricant. The source of alginate can be brown sea-weeds, e.g., *Phaeophyceae* and kelp, and/or bacteria belonging to the genera *Pseudomonas* and *Azotobacter.* The properties of the alginate vary, depending on which species it comes from. Alginates are mainly available in the form of sodium, calcium and ammonium salts. The difference in their monomer structures (mannuronic and guluronic acids) as well as the distribution, proportion and the length of these monomer blocks determine the chemical and physical properties of alginate polymers and oligomers.

Mannuronic and guluronic acids are epimers of each other. Therefore, many physicochemical and biological properties of these two molecules, e.g., molecular formula (C₆H₁₀O₇), molecular weight (194.14 g/mol), boiling point (553.4±50.0 °C at 760 mmHg), and density (1.7±0.1 g/cm³), are the same or highly similar.

Therapeutic properties of mannuronic acid in various animal models were first reported in 2004. The results of these studies revealed the therapeutic efficacy and tolerability of mannuronic acid in *in vitro* and *in vivo* experiments. The potent therapeutic efficacy of mannuronic acid was reported by Mirshafiey et al. 2005 (Mirshafiey A, Cuzzocrea S, Rehm BHA, H. Matsuo H. "M2000: a revolution in pharmacology", Med Sci Monit. 2005. 11(8):153-163). Later studies were carried out to address safety profile, the underlying cellular, molecular and immunological mechanisms and its therapeutic effects at the level of human clinical trials. The results of various studies and clinical trials confirmed that mannuronic acid is very safe and provides for a wide spectrum of therapeutic effects in a variety of diseases, such as rheumatoid arthritis, ankylosing spondylitis, breast cancer, multiple sclerosis, and myelodisplastic syndrome (Fattahi MJ, Jamshidi AR, Mahmoudi M, et al. "Evaluation of the efficacy and safety of β-d-mannuronic acid in patients with ankylosing spondylitis", "A 12-week randomized, placebo-controlled, phase I/II clinical trial", International Immunopharmacology. 2018; 54:112-117; Ahmadi H, Jamshidi AR, Gharibdoost F, et al, "A phase I/II randomized, controlled, clinical trial for assessment of the efficacy and safety of β-D-mannuronic acid in rheumatoid arthritis patients". Inflammopharmacology. 2018; 26(3):737-745; Rezaieyazdi Z, Farooqi A, Soleymani-Salehabadi H, et al. "International multicenter randomized, placebo-controlled phase III clinical trial of β-D-mannuronic acid in rheumatoid arthritis patients". Inflammopharmacology. 2019; 27(5):911-921; Kashefi S, Omranipour R, Mahmoodzadeh H, Ahmadi H, Mirshafiey A. "Clinical improvement of diabetes mellitus type 1 by β-D-mannuronic acid (M2000) in a breast cancer patient - as a case report", Clin Diabetol. 2019; 8(4):227-229; Najafi S, Moghadam NB, Saadat P, et al. "A controlled, randomized phase II clinical trial for efficacy and safety evaluation of mannuronic acid in secondary progressive form of multiple sclerosis", Int J Neurosci. 2022; 132(4):403-412; Ghaderi A, Nodehi SRS, Bakhtiari T, et al, "Mannuronic Acid in Low-Risk and Intermediate-1-Risk Myelodysplastic Syndromes". J Clin Pharmacol. 2020; 60(7):879-888).

The systematic pharmacology and medical investigations on guluronic acid were started in 2013 by Mirshafiey et al, and the results were published in 2015 ("New therapeutic approach by G2013 in experimental model of multiple sclerosis" Afraei S, Azizi G, Zargar SJ, Sedaghat R, Mirshafiey A., in; Acta Neurol Belg. 2015 Sep;115(3):259-66). Furthermore, safety and pharmacotoxicology characteristics of guluronic acid as well as its clinical efficacy on inflammatory diseases have been studied.

These studies showed that not only share these two uronic acids many physicochemical properties, but also have the same or similar biological and medical properties.

Topical formulations in form of gels or creams are known for a variety of pharmaceutical agents and their therapeutic efficacy has been demonstrated. However, many patients show inadequate response to conventional therapy with existing topical gels or creams, for example analgesic or anti-inflammatory formulations that are based on diclofenac or piroxycam as actives. Therefore, it is desirable to provide alternative or improved topical formulations with analgesic and/or anti-inflammatory properties.

### SUMMARY OF THE INVENTION

Surprisingly, it has been found that β-D-mannuronic acid or α-L-guluronic acid, oligomers thereof or pharmaceutically acceptable salts thereof or mixtures of β-D-mannuronic acid and α-L-guluronic acid, oligomers thereof, or pharmaceutically acceptable salts thereof, preferably mannuronate or guluronate or gulumannuronate, can be effectively administered topically in the form of a gel or cream formulation, e.g., as an anti-inflammatory gel or cream formulation for topical use or as an analgesic gel or cream formulation for topical use.

Therefore, in a first aspect, the present invention relates to a gel or cream formulation for topical use, the formulation comprising
(i) β-D-mannuronic acid, an oligomer thereof, or a pharmaceutically acceptable salt thereof, or
(ii) α-L-guluronic acid, an oligomer thereof, or a pharmaceutically acceptable salt thereof, or
(iii) a mixture of β-D-mannuronic acid, an oligomer thereof, or a pharmaceutically acceptable salt thereof and α-L-guluronic acid, an oligomer thereof, or a pharmaceutically acceptable salt thereof, preferably gulumannuronate,
wherein the total amount of β-D-mannuronic acid or α-L-guluronic acid, or their mixtures, oligomers thereof, or pharmaceutically acceptable salts thereof ranges from 0.5 g to 40 g based on 100 g gel or cream formulation.

In various embodiments,
(i) the β-D-mannuronic acid, an oligomer thereof, or a pharmaceutically acceptable salt thereof is β-D-mannuronate, preferably selected from the group consisting of sodium β-D-mannuronate, potassium β-D-mannuronate, magnesium β-D-mannuronate, calcium β-D-mannuronate or ammonium β-D-mannuronate and combinations thereof, more preferably sodium β-D-mannuronate; and/or
(ii) the α-L-guluronic acid, an oligomer thereof, or a pharmaceutically acceptable salt thereof is α-L-guluronate, preferably selected from the group consisting of sodium α-L-guluronate, potassium α-L-guluronate, magnesium α-L-guluronate, calcium α-L-guluronate or ammonium α-L-guluronate and combinations thereof, more preferably sodium α-L-guluronate.

In embodiments where a mixture of β-D-mannuronic acid and α-L-guluronic acid, the respective oligomers thereof, or the respective pharmaceutically acceptable salts thereof is used, the active (pharmaceutical) agent may be gulumannuronate, preferably
wherein β-D-mannuronic acid and α-L-guluronic acid, oligomers thereof, or pharmaceutically acceptable salts thereof are mixed and preferably selected from the group consisting of sodium, or potassium, or magnesium, or calcium, or ammonium β-D-mannuronate and/or α-L-guluronate and combinations thereof, most preferably sodium β-D-mannuronate and/or sodium α-L-guluronate, or
wherein the mixture of β-D-mannuronic acid and α-L-guluronic acid, oligomers thereof, or pharmaceutically acceptable salts thereof, preferably gulumannuronate, is alginate hydrolysate (powder).

In various embodiments, the total amount of
(i) β-D-mannuronic acid, an oligomer thereof, or a pharmaceutically acceptable salt thereof, or
(ii) α-L-guluronic acid, an oligomer thereof, or a pharmaceutically acceptable salt thereof, or
(iii) the mixture of β-D-mannuronic acid and α-L-guluronic acid, oligomers thereof, or pharmaceutically acceptable salts thereof,
in the gel or cream formulation is 0.5 g to 35 g/100 g gel or cream, preferably 1 g to 30 g/100 g gel or cream, more preferably 5 g to 20 g/100 g gel or cream, most preferably 5 g to 10 g/100 g gel or cream.

In various embodiments, the gel or cream formulation can further comprise at least one further pharmaceutical agent and/or at least one further ingredient as described herein.

Preferably, the gel or cream formulation is for anti-inflammatory topical use as described herein. In another embodiment, the gel or cream formulation is an analgesic topical gel or cream formulation as described herein.

In a second aspect, the gel or cream formulation according to the invention is for topical use in a method for treating (or preventing):
(i) inflammation and/or any inflammatory reaction, preferably in joints and/or muscles; and/or
(ii) pain, preferably in joints and/or muscles.

In a third aspect, the present invention relates to a method for preparing a gel or cream formulation as described herein. Preferably, the gel or cream formulation can be a gel or cream formulation for anti-inflammatory topical use as described herein or an analgesic topical gel or cream formulation as described herein.

In various embodiments, the method is a method for preparing a gel formulation, comprising the steps of
(i) providing a (first) solution comprising at least one gel-forming substance and, optionally, at least one preservative;
(ii) providing a (second) solution:
   (a) comprising β-D-mannuronic acid, an oligomer thereof, or a pharmaceutically acceptable salt thereof, preferably β-D-mannuronate, in a total amount of 0.5 g to 40 g, preferably 5 g to 10 g, based on 100 g gel formulation; or
   (b) comprising α-L-guluronic acid, an oligomer thereof, or a pharmaceutically acceptable salt thereof, preferably α-L-guluronate, in a total amount of 0.5 g to 40 g, preferably 5 g to 10 g, based on 100 g gel formulation; or
   (c) comprising a mixture of β-D-mannuronic acid and α-L-guluronic acid, oligomers thereof, or pharmaceutically acceptable salts thereof, preferably gulumannuronate, in a total amount of 0.5 g to 40 g, preferably 5 g to 10 g, based on 100 g gel formulation;
(iii) optionally adjusting the pH of the second solution to pH 1 to 5, preferably 2 to 4, more preferably 3;
(iv) mixing the solutions of i) and ii); and
(v) optionally adjusting the pH to 4 to 8, more preferably 5 to 7, most preferably 5.5 to 6.5.

These and other aspects, embodiments, features, and advantages of the invention become apparent to the person skilled in the art in the following detailed description and claims. Each feature from one aspect of the invention can be used in any other aspect of the invention. Furthermore, the examples contained herein are intended to describe and illustrate the invention, but do not restrict it. In particular, the invention is not limited to these examples.

### DETAILED DESCRIPTION

"At least one", as used herein, means one or more, i.e. 1, 2, 3, 4, 5, 6, 7, 8, 9 or more of the referenced species. Similarly, "one or more", as used herein, relates to at least one and comprises 1, 2, 3, 4, 5, 6, 7, 8, 9 or more. In connection with a given species, the term does not relate to the total number of molecules, but rather to the type of species. "At least one preservative", for example, thus means that one type of preservative or two or more different types of preservatives may be present. In connection with amounts, the term relates to the total amount of the referenced species. In case of preservatives, for example, this means that the given amount is the total amount of all preservatives in the composition/formulation.

As used herein, unless otherwise stated, the singular forms "a", "an", and "the" include plural reference. Thus, for example, a reference to "an oligomer thereof' also includes a plurality of oligomer molecules or oligomer types, e.g. a mixture or combination of various oligomer types. Furthermore, unless otherwise stated, the use of plural forms, e.g. "oligomers thereof' or "pharmaceutical acceptable salts thereof", also includes singular reference such as one type of oligomer or one type of salt.

Numeric values specified without decimal places herein refer to the full value specified with one decimal place, i.e. for example, 99 % means 99.0 %, unless otherwise defined.

The term "about", in connection with a numerical value, refers to a variance of ±10 %, preferably ±5 %, with respect to the given numerical value.

The term "essentially free" within the context of this invention is to be interpreted as the respective compound is contained in the formulation in an amount of less than 5 wt.-%, 4 wt.-%, 3 wt.-%, 2 wt.-%, 1.5 wt.-%, 1 wt.-%, 0.75 wt.-%, 0.5 wt.-%, 0.25 wt.-%, 0.1 wt.-%, 0.01 wt.-%, or 0.001 wt.-% based on the total weight of the formulation, wherein the amounts are respectively more preferred in descending order. For example, 4 wt.-% is more preferred than 5 wt.-% and 3 wt.-% is more preferred than 4 wt.-%.

All percentages given herein in relation to the formulations relate to weight-% (wt.-%) relative to the total weight of the respective formulations, if not explicitly stated otherwise. Numeric ranges specified in the format "from x to y" include the specified values. If multiple preferred numeric ranges are specified in this format, it is understood that all ranges created by combining the different endpoints are also included.

In the following, the term "gel or cream formulation" is used to describe the gel or cream formulation for topical use, preferably for anti-inflammatory topical use and/or for topical analgesic gel formulation. The terms "gel(s)" and "gel formulation(s)" as well as "cream(s)" and "cream formulation(s)" are intended to encompass all viscous formulation types typically used in the field and include pastes, ointments and lotions.

In the context of the present invention, a "cream formulation" may be a two-phase, (semi-solid) dosage form that contains an aqueous phase in addition to a lipid phase (emulsion). In various embodiments, the cream can be a hydrophilic cream or a lipophilic cream.

In the context of the present invention, a "gel formulation" may be a (semi-solid) finely dispersed system with at least two liquid phases or at least one liquid and at least one solid phase, the continuous phase preferably comprising a gel-forming substance. Suitable gel formulations are, for example, water-based gels and alcohol-based gels, without being limited thereto, wherein water-based gel formulations are especially preferred in the present invention.

The viscosity is an important physical property of topical formulations, which can also affect the rate of drug release. Viscosity can be measured in various ways, including vibrating viscometers, capillary viscometers, rotational rheometry, microfluidic rheometers and non-contact rheology. If not defined otherwise, all viscosities mentioned herein are determined by rotational rheometry. Preferably, the formulation according to the invention has a viscosity in the range of 850 cPs to 35000 cPs, preferably in the range of 1000 cPs to 10000 cPs, more preferably in the range of 2500 cPs to 4000 cPs, more preferably in the range of 3000 cPs to 3700 cPs, most preferably in the range of 3100 cPs to 3350 cPs, determined in a laboratory digital Brookfield viscometer (rotational viscometer) at 25°C. In various embodiments, the formulation can be subjected to a steady-state-flow method (0.1-100 s⁻¹) to characterize the flow property and to obtain viscosity values at low (2.0 s⁻¹), medium (20.0 s⁻¹), and high (75.0 s⁻¹) shear rates at 25°C. In preferred embodiments, the shear rate (shear viscosity value) for gel is 75.0 - 85.0 s⁻¹ and for cream is 60.0 - 70.0 s⁻¹, in particular the formulation according to the invention has a viscosity in the range of 850 cPs to 35000 cPs, preferably in the range of 1000 cPs to 10000 cPs, more preferably in the range of 2500 cPs to 4000 cPs, more preferably in the range of 3000 cPs to 3700 cPs, most preferably in the range of 3100 cPs to 3350 cPs, determined in a laboratory digital Brookfield viscometer at 25°C and at a shear rate for gel of 75.0 s⁻¹ - 85.0 s⁻¹ or for cream of 60.0 s⁻¹ - 70.0 s⁻¹.

Beta-D-mannuronic acid has the following formula (I):

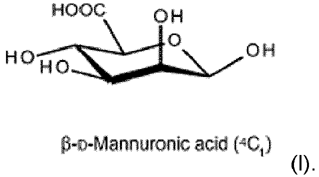

Alpha-L-guluronic acid has the following formula (II):

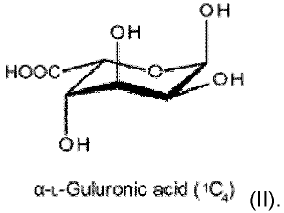

As mannuronic acid and guluronic acid are epimers, they share many physicochemical properties as well as biological and medicinal properties, for example as anti-inflammatory agents with immunosuppressive and immunomodulatory activities. Therefore, in the present invention, all concepts disclosed for β-D-mannuronic acid, an oligomer thereof, or a pharmaceutically acceptable salt thereof are similarly applicable to α-L-guluronic acid, an oligomer thereof, or a pharmaceutically acceptable salt thereof, or to mixtures of both, and *vice versa.*

According to the invention, β-D-mannuronic acid or α-L-guluronic acid or their mixtures can be comprised in the gel or cream formulation in form of pharmaceutically acceptable salts. Preferably, these pharmaceutically acceptable salts are mannuronate, guluronate, or a mixture thereof, more preferably β-D-mannuronate, or α-L-guluronate, or a mixture thereof referred to as "gulumannuronate". The counterion may be selected from sodium, potassium, magnesium, calcium, ammonium and other pharmaceutically acceptable cationic counterions.

Thus, in various embodiments, the gel or cream formulation for topical use can be:
(i) a mannuronate gel or cream formulation; or
(ii) a guluronate gel or cream formulation; or
(iii) a gulumannuronate gel or cream formulation.

In various embodiments, the gel or cream formulation comprising β-D-mannuronic acid, an oligomer thereof, or a pharmaceutically acceptable salt thereof, is a mannuronate gel or cream formulation. Specifically, the β-D-mannuronic acid, an oligomer thereof, or a pharmaceutically acceptable salt thereof can be, in various embodiments, β-D-mannuronate, more preferably selected from the group consisting of sodium β-D-mannuronate, potassium β-D-mannuronate, magnesium β-D-mannuronate, calcium β-D-mannuronate, ammonium β-D-mannuronate and combinations thereof, with sodium β-D-mannuronate being especially preferred.

In various embodiments, β-D-mannuronic acid, preferably β-D-mannuronate, can also be added to the gel or cream formulation in the form of a precursor substance selected from oligomers of β-D-mannuronic acid or β-D-mannuronate, preferably (homo)oligomers of β-D-mannuronate, in particular sodium oligomannuronate, potassium oligomannuronate, magnesium oligomannuronate, calcium oligomannuronate, ammonium oligomannuronate and combinations thereof, especially sodium oligomannuronate.

In various embodiments, β-D-mannuronic acid, an oligomer thereof, or a pharmaceutically acceptable salt thereof, preferably β-D-mannuronate, is selected from the group consisting of sodium β-D-mannuronate, potassium β-D-mannuronate, magnesium β-D-mannuronate, calcium β-D-mannuronate, ammonium β-D-mannuronate, sodium oligomannuronate, potassium oligomannuronate, magnesium oligomannuronate, calcium oligomannuronate, ammonium oligomannuronate and combinations thereof, preferably sodium β-D-mannuronate and/or sodium oligomannuronate.

In various embodiments, the gel or cream formulation comprising α-L-guluronic acid, an oligomer thereof, or a pharmaceutically acceptable salt thereof is a guluronate gel or cream formulation. Specifically, the α-L-guluronic acid, an oligomer thereof, or a pharmaceutically acceptable salt thereof can be, in various embodiments, α-L-guluronate, preferably selected from the group consisting of sodium α-L-guluronate, potassium α-L-guluronate, magnesium α-L-guluronate, calcium α-L-guluronate, ammonium α-L-guluronate and combinations thereof, with sodium α-L-guluronate being especially preferred.

In various embodiments, α-L-guluronic acid, preferably α-L-guluronate, can also be added to the gel or cream formulation in the form of a precursor substance selected from oligomers of α-L-guluronic acid or α-L-guluronate, preferably (homo)oligomers of α-L-guluronate, in particular sodium oligoguluronate, potassium oligoguluronate, magnesium oligoguluronate, calcium oligoguluronate, ammonium oligoguluronate and combinations thereof, especially sodium oligoguluronate.

In various embodiments, α-L-guluronic acid, an oligomer thereof, or a pharmaceutically acceptable salt thereof, preferably α-L-guluronate, is selected from the group consisting of sodium α-L-guluronate, potassium α-L-guluronate, magnesium α-L-guluronate, calcium α-L-guluronate, ammonium α-L-guluronate, sodium oligoguluronate, potassium oligoguluronate, magnesium oligoguluronate, calcium oligoguluronate, ammonium oligoguluronate and combinations thereof, preferably sodium α-L-guluronate and/or sodium oligoguluronate.

In various embodiments, the gel or cream formulation comprising a mixture of β-D-mannuronic acid and α-L-guluronic acid, oligomers thereof, or pharmaceutically acceptable salts thereof, is a gulumannuronate gel or cream formulation. In particular, the mixture of β-D-mannuronic acid and α-L-guluronic acid, oligomers thereof, or pharmaceutically acceptable salts thereof can be a mixture of β-D-mannuronate and α-L-guluronate, preferably selected from the group consisting of sodium β-D-mannuronate, potassium β-D-mannuronate, magnesium β-D-mannuronate, calcium β-D-mannuronate, ammonium β-D-mannuronate and combinations thereof and sodium α-L-guluronate, potassium α-L-guluronate, magnesium α-L-guluronate, calcium α-L-guluronate, ammonium α-L-guluronate and combinations thereof, with a mixture of sodium β-D-mannuronate and sodium α-L-guluronate being especially preferred.

In various embodiments, the mixture of β-D-mannuronic acid and α-L-guluronic acid, preferably gulumannuronate, can also be added to the gel or cream formulation in the form of precursor substances selected from oligomers of β-D-mannuronic acid/β-D-mannuronate and/or α-L-guluronic acid/α-L-guluronate, preferably (homo)oligomers of β-D-mannuronic acid/β-D-mannuronate and/or α-L-guluronic acid/a-L-guluronate, in particular sodium oligomannuronate, potassium oligomannuronate, magnesium oligomannuronate, calcium oligomannuronate, ammonium oligomannuronate, sodium oligoguluronate, potassium oligoguluronate, magnesium oligoguluronate, calcium oligoguluronate, ammonium oligoguluronate and combinations thereof, especially sodium oligomannuronate and/or sodium oligoguluronate.

In various embodiments, β-D-mannuronic acid and/or α-L-guluronic acid, oligomers thereof, or pharmaceutically acceptable salts thereof, preferably gulumannuronate, is selected from the group consisting of sodium β-D-mannuronate, potassium β-D-mannuronate, magnesium β-D-mannuronate, calcium β-D-mannuronate, ammonium β-D-mannuronate, sodium oligomannuronate, potassium oligomannuronate, magnesium oligomannuronate, calcium oligomannuronate, ammonium oligomannuronate, sodium α-L-guluronate, potassium α-L-guluronate, magnesium α-L-guluronate, calcium α-L-guluronate, ammonium α-L-guluronate, sodium oligoguluronate, potassium oligoguluronate, magnesium oligoguluronate, calcium oligoguluronate, ammonium oligoguluronate and combinations thereof, preferably sodium β-D-mannuronate, sodium oligomannuronate, sodium α-L-guluronate, sodium oligoguluronate and combinations thereof.

In particular, β-D-mannuronate oligomers or α-L-guluronate oligomers comprise 2 to 16 monomeric units, preferably 3 to 6 monomeric units, e.g., 3 or 4 or 5 or 6 monomeric units.

In various embodiments, the mixture of β-D-mannuronic acid and α-L-guluronic acid, oligomers thereof, or pharmaceutically acceptable salts thereof, preferably gulumannuronate, can be synthesized from sodium alginate and can be used in the gel or cream formulation in the form of alginate hydrolysate, preferably alginate hydrolysate powder, which is also referred to as gulumannuronate. In particular, alginate hydrolysate (gulumannuronate) is the separated precipitate of sodium alginate comprising mannuronic acid/mannuronate and guluronic acid/guluronate. Therefore, in various embodiments, the gel or cream formulation comprises alginate hydrolysate (gulumannuronate) in concentrations as described herein, preferably 0.5 g to 40 g/100 g gel or cream, more preferably 0.5 g to 35 g/100g gel or cream, more preferably 1 g to 30 g/100 g gel or cream, more preferably 5 g to 20 g/100 g gel or cream, more preferably 5 g to 15 g/100 g gel or cream, most preferably 5 g to 10 g/100g gel or cream.

It is preferred that the mixture of β-D-mannuronic acid and α-L-guluronic acid, oligomers thereof, or pharmaceutically acceptable salts thereof, preferably gulumannuronate, may be (1) a mixture of the single components β-D-mannuronic acid, oligomers thereof, or pharmaceutically acceptable salts thereof, in particular β-D-mannuronate (powder) and α-L-guluronic acid, oligomers thereof, or pharmaceutically acceptable salts thereof, in particular α-L-guluronate (powder); or (2) alginate hydrolysate (powder).

In various embodiments, the total amount of
(i) β-D-mannuronic acid, an oligomer thereof or a pharmaceutically acceptable salt thereof, or
(ii) α-L-guluronic acid, an oligomer thereof, or a pharmaceutically acceptable salt thereof, or
(iii) the mixture of β-D-mannuronic acid or α-L-guluronic acid, oligomers thereof, or pharmaceutically acceptable salts thereof,
in the gel or cream formulation is 0.5 g to 35 g/100 g gel or cream, preferably 1 g to 30 g/100 g gel or cream, more preferably 2 g to 20 g/100 g gel or cream, more preferably 5 g to 20 g/100 g gel or cream such as 5 g/100 g gel or cream, 10 g/100 g gel or cream, 15 g/100 g gel or cream or 20 g/100 g gel or cream, more preferably 5 g to 15 g/100 g gel or cream, most preferably 5 g to 10 g/100 g gel or cream.

In one embodiment, the total amount of β-D-mannuronic acid, an oligomer thereof, or a pharmaceutically acceptable salt thereof in the gel or cream formulation, preferably in the mannuronate gel or cream formulation, is 0.5 g to 40 g/100 g gel or cream, preferably 0.5 g to 35 g/100 g gel or cream, more preferably 1 g to 30 g/100 g gel or cream, more preferably 2 g to 20 g/100 g gel or cream, more preferably 5 g to 20 g/100 g gel or cream such as 5 g/100 g gel or cream, 10 g/100 g gel or cream, 15 g/100 g gel or cream or 20 g/100 g gel or cream, more preferably 5 g to 15 g/100 g gel or cream, most preferably 5 g to 10 g/100 g gel or cream.

In another embodiment, the total amount of α-L-guluronic acid, an oligomer thereof, or a pharmaceutically acceptable salt thereof in the gel or cream formulation, preferably in the guluronate gel or cream formulation, is 0.5 g to 40 g/100 g gel or cream, preferably 0.5 g to 35 g/100 g gel or cream, more preferably 1 g to 30 g/100 g gel or cream, more preferably 2 g to 20 g/100 g gel or cream, more preferably 5 g to 20 g/100 g gel or cream such as 5 g/100 g gel or cream, 10 g/100 g gel or cream, 15 g/100 g gel or cream or 20 g/100 g gel or cream, more preferably 5 g to 15 g/100 g gel or cream, most preferably 5 g to 10 g/100 g gel or cream.

In another embodiment, the total amount of the mixture of β-D-mannuronic acid and α-L-guluronic acid, oligomers thereof, or pharmaceutically acceptable salts thereof in the gel or cream formulation, preferably in the gulumannuronate gel or cream formulation, is 0.5 g to 40 g/100 g gel or cream, preferably 0.5 g to 35 g/100 g gel or cream, more preferably 1 g to 30 g/100 g gel or cream, more preferably 2 g to 20 g/100 g gel or cream, more preferably 5 g to 20 g/100 g gel or cream such as 5 g/100 g gel or cream, 10 g/100 g gel or cream, 15 g/100 g gel or cream or 20 g/100 g gel or cream, more preferably 5 g to 15 g/100 g gel or cream, most preferably 5 g to 10 g/100 g gel or cream.

In various embodiments, the mixture of β-D-mannuronic acid and α-L-guluronic acid, oligomers thereof, or pharmaceutically acceptable salts thereof, preferably (in) the gulumannuronate gel or cream formulation, comprises or consists of
0.1 g to 39.9 g of β-D-mannuronic acid, an oligomer thereof or a pharmaceutically acceptable salt thereof, and
0.1 g to 39.9 g of α-L-guluronic acid, an oligomer thereof, or a pharmaceutically acceptable salt thereof, with the proviso that the total amount of β-D-mannuronic acid, an oligomer thereof, or a pharmaceutically acceptable salt thereof and α-L-guluronic acid, an oligomer thereof, or a pharmaceutically acceptable salt thereof is at least 0.5 g and at most 40 g based on 100 g gel or cream formulation; preferably the total amount of β-D-mannuronic acid, an oligomer thereof, or a pharmaceutically acceptable salt thereof is 0.5 g to 35 g, preferably 1 to 30 g, more preferably 2 g to 20 g, more preferably 5 g to 20 g, more preferably 5 g to 15 g, most preferably 5 g to 10 g, based on 100 g gel or cream formulation, and the total amount of α-L-guluronic acid, an oligomer thereof, or a pharmaceutically acceptable salt thereof is 0.5 g to 35 g, preferably 1 g to 30 g, more preferably 2 g to 20 g, more preferably 5 g to 20 g, more preferably 5 g to 15 g, most preferably 5 g to 10 g, based on 100 g gel or cream formulation. In various embodiments, the total of β-D-mannuronic acid and α-L-guluronic acid, oligomers thereof or pharmaceutically acceptable salts thereof could be: 5.01 g/100 g gel or cream, 10 g/100 g gel or cream, 15 g/100 g gel or cream or 20 g/100 g gel or cream. In one specific embodiment, the mixture comprises or consists of 5 g β-D-mannuronic acid, an oligomer thereof or a pharmaceutically acceptable salt thereof and 5 g α-L-guluronic acid, an oligomer thereof, or a pharmaceutically acceptable salt thereof based on 100 g gel or cream formulation. In various embodiments, the weight ratio of β-D-mannuronic acid, an oligomer thereof, or a pharmaceutically acceptable salt thereof to α-L-guluronic acid, an oligomer thereof, or a pharmaceutically acceptable salt thereof is in the range of from 100:1 to 1:100, such as 10:1 to 1:10 or 5:1 to 1:5, preferably 3:1 to 1:3, 2:1 to 1:2 or about 1.5:1 to 1:1.5, most preferably about 1:1.

The concentration ranges mentioned above for the different gel or cream formulations mean that the gel or cream formulation according to the invention comprises 0.5 to 40 wt.-% of
(i) β-D-mannuronic acid, an oligomer thereof, or a pharmaceutically acceptable salt thereof, or
(ii) α-L-guluronic acid, an oligomer thereof, or a pharmaceutically acceptable salt thereof, or
(iii) the mixture of β-D-mannuronic acid, an oligomer thereof, or a pharmaceutically acceptable salt thereof and α-L-guluronic acid, an oligomer thereof, or a pharmaceutically acceptable salt thereof, preferably 0.5 to 35 wt.-%, more preferably 1 to 30 wt.-%, more preferably 2 to 20 wt.-%, more preferably 5 to 20 wt.-% such as 5 wt.-%, 10 wt.-%, 15 wt.-% or 20 wt-%, more preferably 5 to 15 wt.-%, most preferably 5 to 10 wt.-%, based on the total weight of the gel or cream formulation.

In various embodiments, the gel or cream formulation according to the invention can comprise β-D-mannuronic acid or α-L-guluronic acid, oligomers thereof, or pharmaceutically acceptable salts thereof, or their mixture, preferably β-D-mannuronate or α-L-guluronate or their mixture, together with other, preferably chemically pure, pharmaceutical agents, drugs and/or herbal medicaments.

In various embodiments, the gel or cream formulation comprises at least one further pharmaceutical agent.

In various embodiments, without being limited thereto, the at least one further pharmaceutical agent is selected from analgesic agents, anti-inflammatory agents, and agents with anti-sensitivity properties or combinations thereof.

An analgesic agent is defined, in the context of the present invention, as a drug that eliminates, alleviates or relieves pain. Examples are, without being limited thereto, menthol, lidocaine, camphor, non-steroidal anti-inflammatory drugs (NSAIDs) and opioids.

A suitable anti-inflammatory agent according to the invention is a drug that reduces or eliminates inflammation or inflammatory reaction in the body, e.g., non-steroidal anti-inflammatory drugs (NSAIDs) such as diclofenac, naproxen or ibuprofen, and/or corticosteroids such as cortisol, cortisone, betamethasone without being limited thereto.

Agents with anti-sensitivity properties which can be added to the gel or cream formulation for topical use according to the invention are all anti-allergy or anti-sensitive (desensitizing) agents safe for use in humans. Examples are, without being limited thereto, antihistamines and diphenhydramine HCl.

In various embodiments, the gel or cream formulation comprises at least one further pharmaceutical agent selected from the group consisting of menthol, lidocaine, camphor, non-steroidal anti-inflammatory drugs (NSAIDs), such as diclofenac, naproxen or ibuprofen, corticosteroids, such as cortisol, cortisone or betamethasone, opioids, or anti-allergy or anti-sensitive (desensitizing) agents, such as antihistamines and diphenhydramine HCl.

Therefore, in various embodiments, the gel or cream formulation for topical use according to the invention is an analgesic topical gel or cream formulation, and/or an anti-inflammatory topical gel or cream formulation and/or a gel or cream formulation with anti-sensitivity properties.

In various embodiments, the gel or cream formulation for topical use further comprises caffeic acid or a salt thereof, for example in powder form, preferably in an amount of 0.001 wt.-% to 1 wt.-%, more preferably 0.01 wt.-% to 0.5 wt.-%, more preferably 0.05 wt.-% to 0.3 wt.-%, e.g., 0.1 wt.-% to 0.3 wt.-%, based on the total weight of the gel or cream formulation.

Caffeic acid (3,4-dihydroxycinnamic acid, with the molecular formula C₉H₈O₄) may be found in many plants and foods. For example, coffee is the primary source of caffeic acid in the human diet. However, it can also be found in other food sources such as apples, pears, artichoke and berries. In various embodiments, caffeic acid may have many effects in the body including anti-inflammatory effects. Caffeic acid is commercially available, for example from Sigma Aldrich, or can be isolated from coffee or other food sources.

Therefore, in various embodiments, the gel or cream formulation for topical use comprises
(i) β-D-mannuronic acid, an oligomer thereof, or a pharmaceutically acceptable salt thereof, or
(ii) α-L-guluronic acid, an oligomer thereof, or a pharmaceutically acceptable salt thereof, or
(iii) a mixture of β-D-mannuronic acid, an oligomer thereof, or a pharmaceutically acceptable salt thereof and α-L-guluronic acid, an oligomer thereof, or a pharmaceutically acceptable salt thereof, preferably gulumannuronate,

wherein the total amount of β-D-mannuronic acid or α-L-guluronic acid, or their mixture, oligomers thereof, or pharmaceutically acceptable salts thereof is 0.5 wt.-% to 40 wt.-%, based on the total weight of the gel or cream formulation;
in combination with caffeic acid (powder), preferably in amounts of 0.001 wt.-% to 1 wt.-%, more preferably 0.01 wt.-% to 0.5 wt.-%, more preferably 0.05 wt.-% to 0.3 wt.-%, e.g., 0.1 wt.-% to 0.3 wt.-%, based on the total weight of the gel or cream formulation. It is preferred that such a formulation may be used as an anti-inflammatory topical gel or cream formulation.

In various embodiments, the gel or cream formulation may contain further ingredients such as (one or more) auxiliaries (carriers, skin protectants, disinfectants, preservatives, gel-forming substances, emulsifiers, etc.) known to those skilled in the art and conventionally used in such formulations.

For example, the following auxiliaries may be used in the formulations of the invention, without being limited thereto: particulate carriers (e.g., talc, zinc oxide, starch, starch derivatives, diatomaceous earth); gel-forming substances (e.g., gelatin, tragacanth, cellulose, cellulose derivatives, alginates, polyacrylic acid); cream-forming substances (e.g., lanolin, beeswax, olive oil, arachis oil, liquid paraffin); humectants (e.g., urea, glycerin, propylene glycol), pressure-sensitive polymers (e.g., polyacrylates, and adhesive resins); ointment bases (e.g., petroleum jelly, fats, cellulose derivatives, polyacrylic acid, alginates); emulsifiers (e.g., sodium lauryl sulphate "SLS", stearic acid, lanolin, lecithin, sorbitan esters, monoglycerides); preservatives (e.g., alcohols, glycols, benzoates and their derivatives, e.g., benzalkonium chloride); antioxidants (e.g., butylated hydroxyanisole, vitamin E, ascorbic acid and its derivatives); thickeners (e.g., hydroxypropylmethylcellulose); pH adjusting agents; binders (e.g., polyvinylpyrrolidone, starch, hydroxypropylmethylcellulose, polyethylene glycols); fillers (e.g., microcrystalline cellulose, sorbitol); colorants; flavorings; sweeteners (e.g., sorbitol, aspartame); solvents (e.g., water, ethanol, ethanol-water mixtures); solubilizers (e.g., glycerol, propylene glycol); skin penetration enhancers (e.g., alcohols, terpenes, propylene glycol); plasticizers (e.g., sorbitol, glycerin, phthalic acid esters); wetting agents (e.g., sodium lauryl sulfate, polysorbates); synthetic and natural oils (e.g. medium-chain triglycerides, paraffin, synthetic and/or natural wax, almond sweet oil); propellants for aerosol or foam sprays (e.g. norflurane, cryoflurane, dichlorofluoromethane, trichlorofluoromethane, propane, butane, isobutane, nitrogen).

In various embodiments, the at least one further ingredient is selected from preservatives, alkaline agents, colorants, fragrances, gel-forming substances, cream-forming substances, emulsifiers, thickeners and combinations thereof.

In various embodiments, the gel or cream formulation comprises at least one preservative, preferably selected from the group of anti-bacterial agents, anti-fungal agents, alcohols, glycols, benzoates and/or their derivatives, essential oil or combinations thereof. Preferred preservatives are, e.g., anti-bacterial agents, anti-fungal agents, propylene glycol, butylene glycol, pentylene glycol, ethoxydiglycol, phenoxyethanol, benzyl benzoate, methylisothiazolinone, zinc pyrithione, benzalkonium chloride, sodium benzoate, alcohols, essential oils or derivatives or combinations thereof, without being limited thereto.

It is possible that the gel or cream formulation is free of or essentially free of parabens.

In various embodiments, the gel or cream formulation is free of or essentially free of anti-bacterial agents and/or anti-fungal agents.

In various embodiments, the gel or cream formulation is free of or essentially free of parabens and anti-bacterial agents and anti-fungal agents.

It is preferred that the gel or cream formulation is a gel formulation.

Preferably, the gel formulation according to the invention comprises at least one gel-forming substance and/or at least one thickener.

Preferably, suitable gel-forming substances are selected from pharmaceutical grade components. Suitable polymers, without being limited thereto, are super-absorbent polymers such as those disclosed in WO 92/16245, that include hydrophilic cellulose derivatives which have been partially cross-linked to form a three-dimensional structure. This polymer is a superabsorbent polymer in that it may absorb at least about ten times its own weight of water. The cross-linked structure of the polymer prevents it from dissolving in water but water is easily absorbed into and held within the three-dimensional structure of the polymer to form a gel. Water is lost less rapidly from such a gel than from a solution.

In various embodiments, sodium carboxymethylcellulose, hydroxyethylcellulose, carbomer (polyacrylates/polyacrylic acid; e.g. in powder form) and xanthan gum can advantageously be used as the gel-forming substance.

Especially, the at least one gel-forming substance is selected from carbomers ((homo)polymers of acrylic acid and salts thereof), cellulose, alginates, or derivatives or combinations thereof, typically in a pharmaceutical grade quality.

In particular carbomers (acrylic acid polymers) are used as a suitable basis for many commercial gels, which absorb water readily upon contact and forming a gel.

Carbomers are commercially available, e.g., under the trade names Carbomer 104, Carbomer 934, Carbomer 934 P, Carbomer 940, Carbomer 941, Carbomer 974P, Carbomer 980, Carbomer 1342, Carbomer sodium salt, etc., wherein the numbers refer to the molecule size and the adhesive force.

In preferred embodiments, Carbomer 934 (P) is comprised in a gel formulation according to the invention.

The gel-forming substance, preferably a carbomer, is preferably present in an amount of between about 0.8 wt.-% and about 6.0 wt.-%, more preferably between about 1.5 wt.-% and about 4 wt.-%, more preferably between about 2.0 wt.-% and about 3.0 wt.-%, e.g. 2.5 wt.-%, based on the total weight of the gel formulation.

If the gel or cream formulation is a cream formulation, it preferably comprises at least one cream-forming substance and/or at least one emulsifier.

In various embodiments, the cream-forming substance can be selected from lanolin, beeswax, olive oil, coconut oil, avocado oil, arachis oil, argan oil, and/or petrolatum (petroleum jelly), essential oils, mineral and/or synthetic oils such as liquid paraffin, glycerine, zinc oxide, butyl stearate and diglycol laurate, or combinations thereof.

Suitable emulsifiers can be anionic, cationic, neutral, amphoteric or complex emulsifiers or combinations thereof, and/or synthetic or natural emulsifiers or combinations thereof. In preferred embodiments, the at least one emulsifier is a synthetic and/or natural emulsifier, preferably selected from the group consisting of lecithin, lanolin, sorbitan ester, cetyl alcohol, cetearyl alcohol, stearic acid, glyceryl (mono)stearate, mono-/diglyceride, sodium lauryl sulphate (SLS), benzalkonium chloride, polyoxyl castor oil, carnauba wax, candelilla wax, beeswax or combinations thereof.

Preferred natural emulsifier are, e.g., lanolin, lecithin, carnauba wax, candelilla wax, beeswax or combinations thereof.

In various embodiments, the gel or cream formulation may comprise at least one alkaline agent, for example in the form of a solution, typically to adjust the pH value of the gel or cream formulation.

According to the invention, the gel or cream formulation is administered topically, preferably to a subject in need thereof.

In preferred embodiments, the subject is a mammalian, in particular a human.

In preferred embodiments, the gel or cream formulation according to the invention is for topical use in a method for treating (or preventing)
i) inflammation and/or any inflammatory reaction, preferably in joints and/or muscles; and/or
ii) pain, preferably in joints and/or muscles.

In further preferred embodiments, the gel or cream formulation according to the invention is topically administered to a subject in need thereof in a method for treating (or preventing)
i) inflammation and/or any inflammatory reaction, preferably in joints and/or muscles; and/or
ii) pain, preferably in joints and/or muscles.

In such an embodiment, it is especially preferred that the gel or cream formulation comprises at least one further pharmaceutical agent, preferably at least one analgesic agent, anti-inflammatory agent, or agent with anti-sensitivity properties or combinations thereof, wherein the further pharmaceutical agents are as described herein.

In various embodiments, the gel or cream formulation comprises at least one further pharmaceutical agent, preferably selected from the group consisting of menthol, lidocaine, camphor, non-steroidal anti-inflammatory drugs (NSAIDs) such as diclofenac, naproxen or ibuprofen, corticosteroids such as cortisol, cortisone or betamethasone, opioids, or anti-allergy or anti-sensitive (desensitizing) agents such as antihistamines anddiphenhydramine HCl.

Preferably, the at least one further pharmaceutical agent is a non-steroidal anti-inflammatory drug (NSAID) and/or a corticosteroid, without being limited thereto.

In a further aspect, the present invention relates to a method for preparing a gel or cream formulation according to the invention. The gel or cream formulation is a gel or cream formulation for topical use as described herein.

According to the invention, the resulting gel or cream formulation obtained by the method according to the invention comprises
(i) β-D-mannuronic acid, an oligomer thereof, or a pharmaceutically acceptable salt thereof, preferably β-D-mannuronate, or
(ii) α-L-guluronic acid, an oligomer thereof, or a pharmaceutically acceptable salt thereof, preferably α-L-guluronate, or
(iii) the mixture of β-D-mannuronic acid, an oligomer thereof, or a pharmaceutically acceptable salt thereof, preferably β-D-mannuronate, and α-L-guluronic acid, an oligomer thereof, or a pharmaceutically acceptable salt thereof, preferably α-L-guluronate,
in a total amount of 0.5 g to 40 g based on 100 g gel or cream formulation, preferably in a total amount of 0.5 g to 35 g/100 g gel or cream, more preferably in a total amount of 1 g to 30 g/100g gel or cream, more preferably 2 g to 20 g/100 g gel or cream, most preferably 5 g to 20 g/100 g gel or cream such as 5 g/100g gel or cream, 10 g/100g gel or cream, 15 g/100 g gel or cream or 20 g/100g gel or cream, in particular 5 g to 10 g/100 g gel or cream.

This means that the gel or cream formulation according to the invention comprises 0.5 to 40 wt.-% of
(i) β-D-mannuronic acid, an oligomer thereof, or a pharmaceutically acceptable salt thereof, or
(ii) α-L-guluronic acid, an oligomer thereof, or a pharmaceutically acceptable salt thereof, or
(iii) the mixture of β-D-mannuronic acid, an oligomer thereof, or a pharmaceutically acceptable salt thereof and α-L-guluronic acid, an oligomer thereof, or a pharmaceutically acceptable salt thereof, preferably 0.5 to 35 wt.-%, more preferably 1 to 30 wt.-%, more preferably 2 to 20 wt.-%, more preferably 5 to 20 wt.-% such as 5 wt.-%, 10 wt.-%, 15 wt.-% or 20 wt-%, more preferably 5 to 15 wt.-%, most preferably 5 to 10 wt.-%, based on the total weight of the gel or cream formulation, preferably 100 g gel or cream formulation (final formulation).

In various embodiments, β-D-mannuronic acid, an oligomer thereof, or a pharmaceutically acceptable salt thereof, preferably β-D-mannuronate, and/or α-L-guluronic acid, an oligomer thereof, or a pharmaceutically acceptable salt thereof, preferably α-L-guluronate, is added in the method according to the invention in powder form.

In embodiments in which the resulting gel or cream formulation is gulumannuronate gel or cream formulation comprising a mixture of β-D-mannuronic acid and α-L-guluronic acid, oligomers thereof, or pharmaceutically acceptable salts thereof, preferably β-D-mannuronate and α-L-guluronate, the gel or cream formulation preferably comprises or consists of
0.1 g to 39.9 g of β-D-mannuronic acid, an oligomer thereof, or a pharmaceutically acceptable salt thereof, and
0.1 g to 39.9 g of α-L-guluronic acid, an oligomer thereof, or a pharmaceutically acceptable salt thereof, with the proviso that the total amount of β-D-mannuronic acid, an oligomer thereof, or a pharmaceutically acceptable salt thereof and α-L-guluronic acid, an oligomer thereof, or a pharmaceutically acceptable salt thereof is at least 0.5 g and at most 40 g, based on 100 g gel or cream formulation; preferably the total amount of β-D-mannuronic acid, an oligomer thereof, or a pharmaceutically acceptable salt thereof is 0.5 g to 35 g, more preferably 1 g to 30 g, more preferably 2 g to 20 g, more preferably 5 g to 20 g, more preferably 5 g to 15 g, most preferably 5 g to 10 g, based on 100 g gel or cream formulation, and
the amount of α-L-guluronic acid, an oligomer thereof, or a pharmaceutically acceptable salt thereof is 0.5 g to 35 g, preferably 1 g to 30 g, more preferably 2 g to 20 g, more preferably 5 g to 20 g, more preferably 5 g to 15 g, most preferably 5 g to 10 g, based on 100 g gel or cream formulation. In various embodiments, the total of β-D-mannuronic acid and α-L-guluronic acid, oligomers thereof, or pharmaceutically acceptable salts thereof could be: 5.01 g/100 g gel or cream, 10 g/100 g gel or cream, 15 g/100 g gel or cream or 20 g/100 g gel or cream.

In various embodiments, alginate hydrolysate (powder) can be used (as the mixture of β-D-mannuronic acid, an oligomer thereof, or a pharmaceutically acceptable salt thereof and α-L-guluronic acid, an oligomer thereof, or a pharmaceutically acceptable salt thereof) for gulumannuronate gel or cream preparation. Preferably, alginate hydrolysate (powder) is used in total amounts of 0.5 g to 40 g/100 g gel or cream formulation, more preferably 5 g to 20 g/100 g gel or cream formulation, most preferably 5 g to 10 g/100g gel or cream formulation.

In various embodiments, β-D-mannuronic acid, an oligomer thereof, or a pharmaceutically acceptable salt thereof, preferably β-D-mannuronate, is selected from the group consisting of sodium β-D-mannuronate, potassium β-D-mannuronate, magnesium β-D-mannuronate, calcium β-D-mannuronate, ammonium β-D-mannuronate, and combinations thereof, and/or sodium oligomannuronate, potassium oligomannuronate, magnesium oligomannuronate, calcium oligomannuronate, ammonium oligomannuronate and combinations thereof, with sodium β-D-mannuronate and/or sodium oligomannuronate being especially preferred in the mannuronate gel or cream preparation.

In various embodiments, α-L-guluronic acid, an oligomer thereof, or a pharmaceutically acceptable salt thereof, preferably α-L-guluronate, is selected from the group consisting of sodium α-L-guluronate, potassium α-L-guluronate, magnesium α-L-guluronate, calcium α-L-guluronate, ammonium α-L-guluronate, and combinations thereof, and/or sodium oligoguluronate, potassium oligoguluronate, magnesium oligoguluronate, calcium oligoguluronate, ammonium oligoguluronate and combinations thereof, with sodium α-L-guluronate and/or sodium oligoguluronate being especially preferred in the guluronate gel or cream preparation.

In various embodiments, the mixture of β-D-mannuronic acid and α-L-guluronic acid, oligomers thereof, or pharmaceutically acceptable salts thereof, preferably β-D-mannuronate and α-L-guluronate, are selected from the group consisting of sodium or potassium or magnesium or calcium or ammonium β-D-mannuronate and sodium or potassium or magnesium or calcium or ammonium α-L-guluronate and combinations thereof, and/or their oligomers, preferably their homooligomers consisting of sodium or potassium or magnesium or calcium or ammonium oligomannuronate and sodium or potassium or magnesium or calcium or ammonium oligoguluronate and combinations thereof, with sodium β-D-mannuronate, sodium α-L-guluronate, sodium oligomannuronate and sodium oligoguluronate, and combination thereof being preferred in the gulumannuronate gel or cream preparation.

In particular, oligomannuronate or oligoguluronate comprise 2 to 16 β-D-mannuronic acid or α-L-guluronic acid monomers, preferably 3 to 6 β-D-mannuronic acid or α-L-guluronic acid monomers, e.g., 3 or 4 or 5 or 6 β-D-mannuronic acid or α-L-guluronic acid monomers.

In preferred embodiments, the gel or cream formulation obtained by the method according to the invention may comprise at least one further pharmaceutical agent, preferably selected from analgesic agents, anti-inflammatory agents and/or agents with anti-sensitivity properties as described above.

Thus, in various embodiments, the method according to the invention is a method for preparing an analgesic gel or cream formulation, an anti-inflammatory gel or cream formulation and/or a gel or cream formulation with anti-sensitivity properties.

It is further possible that the gel or cream formulation obtained by the method according to the invention comprises at least one further ingredient such as one or more auxiliaries as described herein.

Preferably, the method is for preparing a gel formulation according to the invention, wherein the method comprises the steps of
(i) providing a (first) solution comprising at least one gel-forming substance and, optionally, at least one preservative;
(ii) providing a (second) solution:
   (a) comprising β-D-mannuronic acid, an oligomer thereof, or a pharmaceutically acceptable salt thereof, preferably β-D-mannuronate, in a total amount of 0.5 g to 40 g, preferably 5 g to 10 g, based on 100 g gel formulation; or
   (b) comprising α-L-guluronic acid, an oligomer thereof, or a pharmaceutically acceptable salt thereof, preferably α-L-guluronate, in a total amount of 0.5 g to 40 g, preferably 5 g to 10 g, based on 100 g gel formulation; or
   (c) comprising a mixture of β-D-mannuronic acid and α-L-guluronic acid, oligomers thereof, or pharmaceutically acceptable salts thereof, preferably gulumannuronate, in a total amount of 0.5 g to 40 g, preferably 5 g to 10 g, based on 100 g gel formulation;
(iii) optionally adjusting the pH of the second solution to pH 1 to 5, preferably 2 to 4, more preferably 3;
(iv) mixing the solutions of i) and ii); and
(v) optionally adjusting the pH to 4 to 8, more preferably 5 to 7, most preferably 5.5 to 6.5.

In various, preferred embodiments, the (first) solution of step i) is an aqueous solution. It is preferred that the at least one gel-forming substance and, optionally, the at least one preservative, is/are dissolved in/mixed with the water, preferably deionized water, to obtain the (first) solution.

Preferably, the gel-forming substance is a polyacrylic acid (carbomer), cellulose, alginate or a derivative or a combination thereof, preferably a (homo)polymer of acrylic acid (such as a carbomer).

It is further preferred that the final concentration of the at least one gel-forming substance, preferably polyacrylic acid (carbomer), in the gel formulation, is between about 0.8 wt.-% and about 6.0 wt.-%, preferably between about 1.5 wt.-% and about 4 wt.-%, more preferably between about 2.0 wt.-% and about 3.0 wt.-%, e.g. about 2.5 wt.-%, based on the total weight of the gel formulation, preferably based on 100 g gel formulation.

In various preferred embodiments, the (second) solution of step ii) is an aqueous solution. It is further preferred that the β-D-mannuronic acid, an oligomer thereof, or a pharmaceutically acceptable salt thereof, preferably β-D-mannuronate for mannuronate gel preparation, or α-L-guluronic acid, an oligomer thereof, or a pharmaceutically acceptable salt thereof, preferably α-L-guluronate for guluronate gel preparation, or the mixture of β-D-mannuronic acid and α-L-guluronic acid, oligomers thereof, or pharmaceutically acceptable salts thereof, preferably β-D-mannuronate and α-L-guluronate, in particular gulumannuronate, for gulumannuronate gel preparation is dissolved in water, preferably deionized water, to obtain the (second) solution. The concentrations are preferably as described above for the general method for preparing a gel or cream formulation, such that the concentrations in the final formulation are as described herein. In various embodiments, alginate hydrolysate (powder) can be used as gulumannuronate (as the mixture of β-D-mannuronic acid, an oligomer thereof, or a pharmaceutically acceptable salt thereof and α-L-guluronic acid, an oligomer thereof, or a pharmaceutically acceptable salt thereof) for gulumannuronate gel preparation and is dissolved in water, preferably deionized water, to obtain the (second) solution. Preferably, alginate hydrolysate (powder) is added in amounts such that a concentration of 0.5 g to 40 g/100 g gel formulation, more preferably 5 g to 20 g/100 g gel formulation, most preferably 5 g to 10 g/100g gel formulation is obtained.

After preparation of the solutions of step i) and ii), the solution of step ii) is mixed with the solution of step i).

In various embodiments, before and/or after mixing, an alkaline agent solution is added to at least one of the solutions, preferably to the (second) solution of step ii) or to the gel formulation after mixing to adjust the pH. If the gel is a water-based gel, the alkaline agent solution is preferably a NaOH solution.

In preferred embodiments, an alkaline agent solution, preferably a NaOH solution, is added to the dissolved mannuronic acid, an oligomer thereof, or a pharmaceutically acceptable salt thereof, or guluronic acid, an oligomer thereof, or a pharmaceutically acceptable salt thereof, or the mixture of mannuronic acid and guluronic acid, oligomers thereof, or pharmaceutically acceptable salts thereof of step ii) to adjust the pH, preferably to a pH of 1 to 5, more preferably 2 to 4, e.g. to a pH of 3.

Preferably, after mixing of the solutions of steps i) and ii), the pH is adjusted to 4 to 8, more preferably 5 to 7, most preferably 5.5 to 6.5.

In various embodiments, the viscosity of the resulting solution (or of the final gel formulation) is adjusted to in a range of 850 cPs to 35000 cPs, preferably 1000 cPs to 10000 cPs, more preferably 2500 cPs to 4000 cPs, more preferably 3000 cPs to 3700, most preferably 3100 cPs to 3350 cPs determined in a laboratory digital Brookfield viscometer (rotational viscometer) at 25°C. In various embodiments, the formulation was subjected to a steady-state-flow method (0.1-100 s⁻¹) to characterize the flow property and to obtain viscosity values at low (2.0 s⁻¹), medium (20.0 s⁻¹), and high (75.0 s⁻¹) shear rates at 25°C. In preferred embodiments, the shear rate (shear viscosity value) for gel is 75.0 - 85.0 s⁻¹, in particular the viscosity of the resulting solution is adjusted to in a range of 850 cPs to 35000 cPs, preferably in the range of 1000 cPs to 10000 cPs, more preferably in the range of 2500 cPs to 4000 cPs, more preferably in the range of 3000 cPs to 3700 cPs, most preferably in the range of 3100 cPs to 3350 cPs, determined in a laboratory digital Brookfield viscometer at 25°C and at a shear rate of 75.0 s⁻¹ - 85.0 s⁻¹.

In preferred embodiments, the solution of step i) and/or the solution of step ii) may comprise at least one further pharmaceutical agent, preferably selected from analgesic agents, anti-inflammatory agents and/or agents with anti-sensitivity properties as described above.

Therefore, in various embodiments, the method according to the invention is a method for preparing an analgesic gel formulation, an anti-inflammatory gel formulation and/or a gel formulation with anti-sensitivity properties.

It is further possible that the (first) solution of step i) and/or the (second) solution of step ii) comprise(s) at least one further ingredient such as one or more auxiliaries as described herein.

The further pharmaceutical agents and/or the further ingredient (auxiliary) may also be comprised in a further solution, e.g., in a third or fourth solution that may be added in later steps.

Preferably, further solutions, if present, are also aqueous solutions as described above. In various embodiments, the pH can be adjusted, preferably as described above, before mixing with the solution of step i) and/or ii).

In various embodiments, further solutions, if present, are mixed with the solutions of step i) and/or with the solution of step ii) or the mixture of both. Afterwards, preferably the pH is adjusted to pH 4 to 8, more preferably 5 to 7, most preferably 5.5 to 6.5.

Additional water can be added to the resulting solution to obtain a gel formulation according to the invention. This step is an optional step to adjust the final concentrations of the ingredients of the gel formulations and/or to adapt the consistence and/or viscosity of the gel formulation, for example.

In various embodiments, the method comprises the steps of
(i) providing a (first) solution comprising at least one gel-forming substance and, optionally, at least one further pharmaceutical agent and/or at least one further ingredient, e.g., at least one preservative;
(ii) providing a (second) solution comprising
   (a) β-D-mannuronic acid, an oligomer thereof, or a pharmaceutically acceptable salt thereof, preferably β-D-mannuronate, in a total amount of 0.5 g to 40 g, preferably 0.5 g to 35 g, more preferably 1 g to 30 g, more preferably 2 g to 20 g, more preferably 5 g to 20 g, more preferably 5 g to 15 g, most preferably 5 g to 10 g, based on 100 g gel formulation; or
   (b) α-L-guluronic acid, an oligomer thereof, or a pharmaceutically acceptable salt thereof, preferably α-L-guluronate, in a total amount of 0.5 g to 40 g, preferably 0.5 g to 35 g, more preferably 1 g to 30 g, more preferably 2 g to 20 g, more preferably 5 g to 20 g, more preferably 5 g to 15 g, most preferably 5 g to 10 g, based on 100 g gel formulation; or
   (c) a mixture of β-D-mannuronic acid and α-L-guluronic acid, oligomers thereof, or pharmaceutically acceptable salts thereof, preferably gulumannuronate, in a total amount of 0.5 g to 40 g, preferably 0.5 g to 35 g, more preferably 1 g to 30 g, more preferably 2 g to 20 g, more preferably 5 g to 20 g, more preferably 5 g to 15 g, most preferably 5 g to 10 g, based on 100 g gel formulation;
   and optionally at least one further pharmaceutical agent and/or at least one further ingredient;
(iii) optionally providing at least one further solution comprising at least one further pharmaceutical agent and/or at least one further ingredient;
(iv) optionally adjusting the pH of the second solution to pH 1 to 5, preferably 2 to 4, more preferably 3;
(v) mixing the solutions of step i) and step ii), and, optionally, step iii);
(vi) optionally adjusting the pH of the resulting solution to 4 to 8, more preferably 5 to 7, most preferably 5.5 to 6.5;
(vii) optionally adjusting the viscosity of the resulting solution to 850 cPs to 35000 cPs, preferably 2500 cPs to 4000 cPs, more preferably 3000 cPs to 3700, most preferably 3100 cPs to 3350 cPs, determined in a laboratory digital Brookfield viscometer (rotational viscometer) at 25°C, preferably at a shear rate of 75.0 s⁻¹ - 85.0 s⁻¹;
(viii) optionally adding water to the resulting solution to obtain a gel formulation, preferably a gel formulation as described herein.

In various embodiments, the method according to the invention is a method for preparing a cream formulation according to the invention.

In various embodiments, in a (topical) cream formulation, ingredients are dissolved or dispersed in either a water-in-oil (W/O) emulsion or an oil-in-water (O/W) emulsion.

Water-in-oil (W/O) or oil-in-water (O/W) emulsions are known to those skilled in the art and can be routinely prepared. Preferably, the (topical) cream formulation has a higher content of oily/lipophilic substances than a gel. In one embodiment, the cream formulation according to the invention comprises the water-base in about 60-75 % by weight and the oil-base in about 20-30 % by weight, based on the total amount of cream formulation.

According to the invention, the cream formulation may comprise
(a) β-D-mannuronic acid, an oligomer thereof, or a pharmaceutically acceptable salt thereof, preferably β-D-mannuronate, in a total amount of 0.5 g to 40 g, preferably 5 g to 10 g, based on 100 g cream formulation; or
(b) α-L-guluronic acid, an oligomer thereof, or a pharmaceutically acceptable salt thereof, preferably α-L-guluronate, in a total amount of 0.5 g to 40 g, preferably 5 g to 10 g, based on 100 g cream formulation; or
(c) a mixture of β-D-mannuronic acid and α-L-guluronic acid, oligomers thereof, or pharmaceutically acceptable salts thereof, preferably gulumannuronate, in a total amount of 0.5 g to 40 g, preferably 5 g to 10 g, based on 100 g cream formulation. In various embodiments, alginate hydrolysate (powder) can be added as gulumannuronate (as the mixture of β-D-mannuronic acid, an oligomer thereof or a pharmaceutically acceptable salt thereof and α-L-guluronic acid, an oligomer thereof, or a pharmaceutically acceptable salt thereof) for gulumannuronate cream preparation. Preferably, alginate hydrolysate (powder) is added in amounts of 0.5 g to 40 g/100 g cream formulation, more preferably 5 g to 20 g/100 g cream formulation, most preferably 5 g to 10 g/100g cream formulation.

Further ingredients include, for example, at least one cream-forming substance, at least one emulsifier, at least one plasticizer, e.g. cetyl stearyl 2-ethyl hexanoate, and/or at least one preservative as described above.

Preferably, the method is for preparing a cream formulation according to the invention, wherein the method comprises the steps of
(i) providing a (first) composition comprising at least one cream-forming substance and, optionally, at least one emulsifier, at least one plasticizer and/or at least one preservative;
(ii) providing a (second) composition:
   (a) comprising β-D-mannuronic acid, an oligomer thereof, or a pharmaceutically acceptable salt thereof, preferably β-D-mannuronate, in a total amount of 0.5 g to 40 g, preferably 5 g, based on 100 g cream formulation; or
   (b) comprising α-L-guluronic acid, an oligomer thereof, or a pharmaceutically acceptable salt thereof, preferably α-L-guluronate, in a total amount of 0.5 g to 40 g, preferably 5 g, based on 100 g cream formulation; or
   (c) comprising a mixture of β-D-mannuronic acid and α-L-guluronic acid, oligomers thereof, or pharmaceutically acceptable salts thereof, preferably gulumannuronate, in a total amount of 0.5 g to 40 g, preferably 5 g, based on 100 g cream formulation;
(iii) optionally adding at least one further pharmaceutical agent and/or at least one further ingredient, optionally in the first or second step or a further step;
(iv) optionally adjusting the pH of the second solution to pH 1 to 5, preferably 2 to 4, more preferably 3;
(v) mixing the solutions of i) and ii); and
(vi) optionally adjusting the pH to 4 to 8, more preferably 5 to 7, most preferably 5.5 to 6.5.

Preferably, in step i), a semi-solid composition or emulsion, preferably a water-in-oil (W/O) emulsion or an oil-in-water (O/W) emulsion, is prepared. In various embodiments, the at least one cream-forming substance and, optionally, the at least one emulsifier, the at least one plasticizer and/or the at least one preservative is/are dissolved/mixed in/with water, preferably deionized water.

Further suitable ingredients include, for example, at least one thickener as described herein and/or at least one (organic) solvent such as propylene glycol.

The additional pharmaceutical agents are preferably those as described above. In various embodiments, the method according to the invention is a method for preparing an analgesic cream formulation, an anti-inflammatory cream formulation and/or a cream formulation with anti-sensitivity properties.

In various embodiments, the resulting gel or cream formulation can be sterilised. Suitable methods for sterilising the formulation include, by way of example, heat/steam treatment, radiation and aseptic production.

The gel or cream formulation according to the invention can be filled or packaged in any suitable packaging, e.g., tubes, films or other packages. These may be made of any suitable material, such as aluminium-based tubes or low melting point polyolefin packages, such as polyethylene packages.

All embodiments and examples described herein for the gel or cream formulation for topical use according to the invention also apply to the gel or cream formulation for topical use in a method for treating inflammation and/or any inflammatory reaction, and/or pain, and to the method for preparing said gel or cream formulation and *vice versa.*

Other embodiments are within the following non-limiting examples.

### Examples

### 1. Method of preparation of mannuronic acid, guluronic acid and gulumannuronate powders from sodium alginate.

Mannuronic acid, guluronic acid and gulumannuronate were synthesized according to the GMP criteria of the WHO for substance production.

To synthesize "β-D-mannuronic acid", "α-L-guluronic acid" and "gulumannuronate", the alginic acid sodium salt (sodium alginate) was used. In a first step, sodium alginate (100 g) was dissolved gently in 1500 ml H₂SO₄ 20% at 0 °C. The solution was thoroughly stirred at room temperature and was then heated to 85 °C until its color changed from a light cream color to light brown. The hydrolysate was allowed to cool to room temperature and the precipitate separated by centrifugation (3700 g). This precipitate can be used as gulumannuronate (alginate hydrolysate). The precipitate was re-dissolved by neutralization using 1 M Na₂CO₃ solution. This solution was then adjusted to pH 2.85 using 0.5 M HCl and the precipitate again separated by centrifugation (3700 g). The precipitate was collected and washed once with distilled water. This precipitate (α-L-guluronic acid) was spread and dried in petri dishes to yield α-L-guluronic acid in powder form. Said powder can be used in guluronate gel or cream preparation and/or in the mixture of β-D-mannuronic acid and α-L-guluronic acid for gulumannuronate gel or cream preparation. The remaining supernatant of the L-guluronic acid precipitate was collected and adjusted to pH 1.0 using 0.5 M HCl. Following centrifugation (3700 g), the precipitate was collected and washed once with distilled water. The obtained precipitate (β-D-mannuronic acid) was spread and dried in petri dishes to yield β-D-mannuronic acid in powder form. This may be used in mannuronate gel or cream preparation and/or in the mixture of β-D-mannuronic acid with α-L-guluronic acid for gulumannuronate gel or cream preparation. The characteristics of α-L-guluronic acid and β-D-mannuronic acid and their purity were validated by Fourier Transform Infrared (FT-IR) spectroscopy and Carbon-13 Nuclear Magnetic Resonance (13C-NMR) spectroscopy.

The provided powders (mannuronic acid) and (guluronic acid) were stored at room temperature (22 - 26 °C) in a dry place under sterile conditions in order to prepare the topical gel or cream.

### 2. Method of preparation of mannuronate, guluronate and gulumannuronate topical gels

I): In the first stage, the preferred preservative agent(s) is/are dissolved in 70 ml boiling deionized water and then 2.5 g Carbomer 934 P is added to obtain a clear / transparent solution. This solution is then cooled (Solution 1).
II): In a separate glass beaker:
   IIa): for mannuronate gel preparation, 10 g mannuronic powder is added to 20 ml deionized water and then adjusted to pH 3 by adding NaOH 30% to completely dissolve the mannuronate (Solution 2a).
   IIb): for guluronate gel preparation, 10 g guluronic powder is added to 20 ml deionized water and then adjusted to pH 3 by adding NaOH 30% to completely dissolve the guluronate (Solution 2b).
   IIc): for gulumannuronate gel preparation, 5 g mannuronic powder and 5 g guluronic powder are added to 20 ml deionized water and adjusted to pH 3 by adding NaOH 30% to dissolve the powders (Solution 2c). An alternative way for gulumannuronate gel preparation is to use the first precipitate during the sodium alginate hydrolysis process. For this purpose, 10 g gulumannuronte (alginate hydrolysate) powder is added to 20 ml deionized water and is dissolved by adding NaOH 30% until pH 3 (Solution 2c).
III): After adding (Solution 2) to (Solution 1) and obtaining a clear solution, the pH is slowly adjusted to 5.5 - 6 (Solution 3).
IV): Deionized water is added to (Solution 3) up to a total (final) weight of 100 g.
V): The synthesized mannuronate topical gel (using solution 2a) and guluronate topical gel (using solution 2b) and gulumannuronate topical gel (using solution 2c) can be packaged under sterile conditions and then stored.

## Claims

1. A gel or cream formulation for topical use, the formulation comprising
(i) β-D-mannuronic acid, an oligomer thereof, or a pharmaceutically acceptable salt thereof, or
(ii) α-L-guluronic acid, an oligomer thereof, or a pharmaceutically acceptable salt thereof, or
(iii) a mixture of β-D-mannuronic acid, an oligomer thereof, or a pharmaceutically acceptable salt thereof and α-L-guluronic acid, an oligomer thereof, or a pharmaceutically acceptable salt thereof, preferably gulumannuronate,
wherein the total amount of β-D-mannuronic acid or α-L-guluronic acid, or their mixture, oligomers thereof, or pharmaceutically acceptable salts thereof is 0.5 g to 40 g based on 100 g gel or cream formulation.

2. The gel or cream formulation according to claim 1, wherein the formulation has a viscosity of 850 cPs to 35000 cPs, preferably 2500 cPs to 4000 cPs, more preferably 3000 cPs to 3700, most preferably 3100 cPs to 3350 cPs determined in a laboratory digital Brookfield viscometer (rotational viscometer) at 25°C, preferably at a shear rate for gel of 75.0 s⁻¹ - 85.0 s⁻¹ or for cream of 60.0 s⁻¹ - 70.0 s⁻¹.

3. The gel or cream formulation according to claim 1 or 2, wherein
(i) the β-D-mannuronic acid, an oligomer thereof, or a pharmaceutically acceptable salt thereof is β-D-mannuronate, preferably selected from the group consisting of sodium β-D-mannuronate, potassium β-D-mannuronate, magnesium β-D-mannuronate, calcium β-D-mannuronate, or ammonium β-D-mannuronate and combinations thereof, more preferably sodium β-D-mannuronate; or
(ii) the α-L-guluronic acid, an oligomer thereof, or a pharmaceutically acceptable salt thereof is α-L-guluronate, preferably selected from the group consisting of sodium α-L-guluronate, potassium α-L-guluronate, magnesium α-L-guluronate, calcium α-L-guluronate, or ammonium α-L-guluronate and combinations thereof, more preferably sodium α-L-guluronate; or
(iii) the mixture of β-D-mannuronic acid and α-L-guluronic acid, oligomers thereof, or pharmaceutically acceptable salts thereof, preferably gulumannuronate, is selected from the group consisting of sodium, or potassium, or magnesium, or calcium, or ammonium β-D-mannuronate and α-L-guluronate and combinations thereof, preferably sodium β-D-mannuronate and/or sodium α-L-guluronate; or
(iv) the mixture of β-D-mannuronic acid and α-L-guluronic acid, oligomers thereof, or pharmaceutically acceptable salts thereof, preferably gulumannuronate, is alginate hydrolysate (powder).

4. The gel or cream formulation according to any one of claims 1 to 3, wherein β-D-mannuronic acid and/or α-L-guluronic acid, oligomers thereof, or pharmaceutically acceptable salts thereof are selected from oligomers of β-D-mannuronate and/or α-L-guluronate.

5. The gel or cream formulation according to claim 4, wherein the oligomer of β-D-mannuronate and/or α-L-guluronate
(i) is the homooligomer of β-D-mannuronate and/or the homooligomer of α-L-guluronate;
(ii) is selected from the group consisting of sodium, or potassium, or magnesium, or calcium, or ammonium oligomannuronate and oligoguluronate and combinations thereof, preferably sodium oligomannuronate and/or sodium oligoguluronate; and/or
(iii) comprises or consists of 2 to 16 β-D-mannuronic acid monomers and/or 2 to 16 α-L-guluronic acid monomers.

6. The gel or cream formulation according to any one of claims 1 to 5, wherein the total amount of
(i) β-D-mannuronic acid, an oligomer thereof, or a pharmaceutically acceptable salt thereof, or
(ii) α-L-guluronic acid, an oligomer thereof, or a pharmaceutically acceptable salt thereof, or
(iii) the mixture of β-D-mannuronic acid or α-L-guluronic acid, oligomers thereof, or pharmaceutically acceptable salts thereof,
in the gel or cream formulation is 1 g to 30 g/100 g gel or cream, more preferably 5 g to 20 g/100 g gel or cream, most preferably 5 g to 10 g/100 g gel or cream.

7. The gel or cream formulation according to claim 6, wherein the mixture of β-D-mannuronic acid and α-L-guluronic acid, oligomers thereof, or pharmaceutically acceptable salts thereof, preferably the gulumannuronate formulation, comprises or consists of 0.1 g to 39.9 g of β-D-mannuronic acid, an oligomer thereof, or a pharmaceutically acceptable salt thereof, and 0.1 g to 39.9 g of α-L-guluronic acid, an oligomer thereof, or a pharmaceutically acceptable salt thereof, with the proviso that the total amount of β-D-mannuronic acid, an oligomer thereof, or a pharmaceutically acceptable salt thereof and α-L-guluronic acid, an oligomer thereof, or a pharmaceutically acceptable salt thereof is at least 0.5 g and at most 40 g, based on 100 g gel or cream formulation; preferably
the total amount of β-D-mannuronic acid, an oligomer thereof, or a pharmaceutically acceptable salt thereof is 0.5 g to 35 g, preferably 1 g to 30 g, more preferably 2 g to 20 g, more preferably 5 g to 15 g, most preferably 5 g to 10 g, based on 100 g gel or cream formulation, and
the total amount of α-L-guluronic acid, an oligomer thereof, or a pharmaceutically acceptable salt thereof is 0.5 g to 35 g, preferably 1 g to 30 g, more preferably 2 g to 20 g, more preferably 5 g to 15 g, most preferably 5 g to 10 g, based on 100 g gel or cream formulation.

8. The gel or cream formulation according to any one of claims 1 to 7, wherein the gel or cream formulation comprises at least one further pharmaceutical agent, preferably selected from analgesic agents, anti-inflammatory agents and agents with anti-sensitivity properties or combinations thereof.

9. The gel or cream formulation according to any one of claims 1 to 8, wherein the gel or cream formulation further comprises at least one further ingredient selected from preservatives, alkaline agents, colorants, fragrances, gel-forming substances, emulsifiers and combinations thereof.

10. The gel or cream formulation according to claim 9, wherein the at least one preservative is selected from the group of anti-bacterial agents, anti-fungal agents, alcohols, glycols, benzoates and/or their derivatives, essential oil or combinations thereof.

11. The gel or cream formulation according to any one of claims 1 to 10, wherein the gel or cream formulation is free of or essentially free of parabens and/or anti-bacterial agents and/or anti-fungal agents.

12. The gel formulation according to any one of claims 9 to 11, wherein the at least one gel-forming substance is polyacrylic acid (carbomer), cellulose, alginate or their derivatives or combinations thereof, preferably a (homo)polymer of acrylic acid (carbomer).

13. The cream formulation according to any one of claims 9 to 11, wherein the at least one emulsifier is a synthetic or natural emulsifier, preferably selected from the group consisting of lecithin, lanolin, sorbitan ester, cetyl alcohol, cetearyl alcohol, stearic acid, glyceryl (mono)stearate, mono-/diglyceride, sodium lauryl sulphate (SLS), benzalkonium chloride, polyoxyl castor oil, carnauba wax, candelilla wax, beeswax and combinations thereof.

14. The gel or cream formulation according to any one of claims 1 to 13 for topical use in a method for treating (or preventing)
(i) inflammation and/or any inflammatory reaction, preferably in joints and/or muscles; and/or
(ii) pain, preferably in joints and/or muscles.

15. A method for preparing a gel or cream formulation according to any one of claims 1 to 13, wherein the method is preferably for preparing a gel formulation, wherein the method comprises the steps of
(i) providing a (first) solution comprising at least one gel-forming substance and, optionally, at least one preservative;
(ii) providing a (second) solution:
(a) comprising β-D-mannuronic acid, an oligomer thereof, or a pharmaceutically acceptable salt thereof, preferably β-D-mannuronate, in a total amount of 0.5 g to 40 g, preferably 5 g to 10 g, based on 100 g gel formulation; or
(b) comprising α-L-guluronic acid, an oligomer thereof, or a pharmaceutically acceptable salt thereof, preferably α-L-guluronate, in a total amount of 0.5 g to 40 g, preferably 5 g to 10 g, based on 100 g gel formulation; or
(c) comprising a mixture of β-D-mannuronic acid and α-L-guluronic acid, oligomers thereof, or pharmaceutically acceptable salts thereof, preferably gulumannuronate, in a total amount of 0.5 g to 40 g, preferably 5 g to 10 g, based on 100 g gel formulation;
(iii) optionally adjusting the pH of the second solution to pH 1 to 5, preferably 2 to 4, more preferably 3;
(iv) mixing the solutions of i) and ii); and
(v) optionally adjusting the pH to 4 to 8, more preferably 5 to 7, most preferably 5.5 to 6.5.
